(19) Europäisches Patentamt / European Patent Office / Office européen des brevets

(11) **EP 3 711 664 A1**

## (12) EUROPEAN PATENT APPLICATION
published in accordance with Art. 153(4) EPC

(43) Date of publication:
**23.09.2020 Bulletin 2020/39**

(21) Application number: **18879885.4**

(22) Date of filing: **01.10.2018**

(51) Int Cl.:
**A61B 5/0408** (2006.01)    **A61B 5/0478** (2006.01)

(86) International application number:
**PCT/JP2018/036680**

(87) International publication number:
**WO 2019/097860 (23.05.2019 Gazette 2019/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.11.2017   JP 2017221866**

(71) Applicant: **Toyobo Co., Ltd.**
**Osaka-shi, Osaka 530-8230 (JP)**

(72) Inventors:
• **NARUSAWA Haruhiko**
  **Otsu-shi**
  **Shiga 520-0292 (JP)**
• **IRIE Michihiko**
  **Otsu-shi**
  **Shiga 520-0292 (JP)**
• **KONDO Takashi**
  **Otsu-shi**
  **Shiga 520-0292 (JP)**

(74) Representative: **Müller-Boré & Partner**
**Patentanwälte PartG mbB**
**Friedenheimer Brücke 21**
**80639 München (DE)**

(54) **GARMENT FOR BIOLOGICAL INFORMATION MEASUREMENT AND STRETCHABLE LAYERED SHEET**

(57) The present invention provides a stretchable laminate sheet for a garment for measuring biological information. The stretchable laminate sheet has favorable conductivity and stretchability, and has such an enhanced adhesiveness to a substrate as to prevent peeling and disconnection of an electrode that are caused by stress and deformation when the garment is put on, taken off, and washed, while detecting a stable electrical signal. The stretchable laminate sheet of the present invention at least includes a stretchable insulating layer having adhesiveness and a stretchable conductor layer. The stretchable laminate sheet has a 90 degree peel strength between the stretchable insulating layer and the adherend layer of 1.0 N/cm or more.

EP 3 711 664 A1

**Description**

TECHNICAL FIELD

The present invention relates to electrical wires, electrodes, stretchable conductors, and insulating films of garments for measuring biological information. The present invention relates, for example, to a garment for measuring biological information that offers excellent comfort and is capable of measuring a faint electrical signal inside a living body by contact with a skin surface of the living body, and relates to a stretchable laminate sheet that is useful as an electrode and an electrical wire of the garment.

BACKGROUND ART

For measurement of a faint bioelectrical signal inside a living body such as brainwaves, an electrocardiogram and an electromyogram, there have conventionally been an adhesive bioelectrode pad (Patent Document 1) and the like formed from a soft conductive adhesive gel having conductivity. Such an adhesive electrode pad has adhesiveness to skin and has been used for a subject at rest to establish conductivity not to make an electrical noise and signal degradation in the bioelectrical signal.

For continuous detection of a bioelectrical signal from a subject in motion for long hours in his/her daily life, there has also been a bioelectrode attachable to a garment and the like to be utilized for healthcare, disease prediction and the like. Such a bioelectrode and the following ones have been proposed to supersede the adhesive gel electrode: a sealing-layer bioelectrode including chitosan or a chitosan derivative that has adhesiveness to skin (Patent Document 2); a textile knitted fabric bioelectrode having enhanced adhesiveness to skin that comes from its enhanced surface smoothness (Patent Document 3); and a stretchable sheet electrode formed from dried conductive paste that enables stable signal detection through an entire surface of the electrode that adheres to and follows skin (Patent Document 4).

An emphasis is placed on adhesiveness of these electrodes to skin for stable electrical signal detection from a subject even in motion. However, attempting to integrate these electrodes into garments might break the electrodes or peel thereof from the garments owing to stress and deformation that the bioelectrodes would undergo upon a repeated act of putting on and taking off the garments, and repeated washing thereof. For this reason, such an electrode-integrated garment has been difficult to handle in a same manner as a general garment from a perspective of convenience.

PRIOR ART DOCUMENTS

PATENT DOCUMENTS

Patent Document 1: Japanese Unexamined Patent Application Publication No. 09-215668
Patent Document 2: Japanese Unexamined Patent Application Publication No. 2002-035141
Patent Document 3: Japanese Patent No. 06185638
Patent Document 4: WO2016/114339

SUMMARY OF THE INVENTION

TECHNICAL PROBLEM

As mentioned above, the garment with the electrode for the measurement of an electrical signal for long hours in a daily life is needed to adhere to and follow skin for preventing noise contamination and deterioration of the electrical signal in case that a wearer of the garment takes various postures and makes various motions in his/her daily life. In addition, the electrode is needed to have sufficient physical properties and adhesiveness to a garment because the electrode is subjected to stress and deformation when the garment is put on, taken off, and washed.

The present invention has been made with the above-mentioned technical problems as a backdrop. An object of the present invention is to provide a stretchable laminate sheet for a garment for measuring biological information. The stretchable laminate sheet has favorable conductivity and stretchability, and sufficient physical properties and adhesiveness to the garment for prevention of peeling and disconnection of an electrode that are caused by stress and deformation when the garment is put on, taken off, and washed, while detecting a stable electrical signal.

SOLUTIONS TO THE PROBLEMS

That is, the present invention has the following configuration.

[1] A garment for measuring biological information having an electrical wire comprising:

a flexible adherend layer having an elongation at break of 10% or more; and
a stretchable insulating layer having a 90 degree peel strength against the flexible adherend layer of 1.0 N/cm or more and 25.0 N/cm or less.

[2] The garment for measuring biological information according to above [1],
wherein the adherend layer is at least one layer selected from the group consisting of a clothing fabric, an insulating base layer, and a conductor layer.
[3] A stretchable laminate sheet at least comprising:

a stretchable conductor layer used as an electrical wire or a body contact electrode of a garment for measuring biological information; and
a stretchable insulating layer having a 90 degree peel strength against the stretchable conductor layer of 1.0 N/cm or more and 25.0 N/cm or less.

[4] The stretchable laminate sheet according to above [3],
wherein the stretchable laminate sheet has a tensile elastic modulus of 10 MPa or more and 500 MPa or less, and
wherein a load applied to the stretchable laminate sheet at a stretching rate of 10% is 3 N or more and 100 N or less.
[5] The stretchable laminate sheet according to above [3] or [4],
wherein the stretchable conductor layer has a sheet resistance of 300 $\Omega_\square$ or less in the absence of stretching, and
wherein the stretchable conductor layer has a sheet resistance increase ratio at a stretching rate of 10% of less than 10.
[6] The stretchable laminate sheet according to any one of above [3] to [5],
wherein the stretchable insulating layer having adhesiveness comprises a flexible resin,
wherein the conductor layer comprises an electrically conductive composition comprising electrically conductive fine particles and a binder resin,
wherein a value calculated by subtraction of a Hansen solubility parameter value ($\delta$) of the binder resin from a Hansen solubility parameter value ($\delta$) of the flexible resin is -5.0 to +5.0 $(MPa)^{0.5}$, and
wherein a value calculated by subtraction of a hydrogen bond term ($\delta h$) of Hansen solubility parameters of the binder resin from a hydrogen bond term ($\delta h$) of Hansen solubility parameters of the flexible resin is -6.0 to +6.0 $(MPa)^{0.5}$.
[7] The stretchable laminate sheet according to any one of above [3] to [6],
wherein the binder resin has an elastic modulus of 1 GPa or less, and
wherein the binder resin has an elongation at break of 200% or more.
[8] A method for producing the stretchable laminate sheet according to any one of above [3] to [7],
wherein the stretchable laminate sheet is produced by application or printing.
[9] The garment for measuring biological information according to above [1] or [2],
wherein the stretchable laminate sheet according to any one of above [3] to [8] is used as the electrical wire.
[10] The garment for measuring biological information according to above [9],
wherein a 90 degree peel strength between the stretchable laminate sheet and a fabric is 1.0 N/cm or more and 25.0 N/cm or less.

ADVANTAGEOUS EFFECTS OF THE INVENTION

The garment of the present invention is a garment for measuring biological information having an electrical wire. The garment includes a flexible adherend layer having an elongation at break of 10% or more and a stretchable insulating layer having a 90 degree peel strength against the flexible adherend layer of 1.0 N/cm or more and 25.0 N/cm or less. The adherend layer is at least one layer selected from the group consisting of a clothing fabric, an insulating base layer, and a conductor layer. That is, the present invention achieves the garment for measuring biological information that has an insulating property for the electrical wire and has sufficient adhesiveness between the insulating layer and the adherend enabling the garment to sufficiently withstand stress and keep enough electrical insulation property as for example, the garment is put on, taken off, and washed.
The present invention is applied to a garment for measuring biological information that is produced in such a manner as to form an electrical wire, an electrode, or the like on its garment body. The application of the present invention

**EP 3 711 664 A1**

is preferably in a form of the stretchable laminate sheet in which the conductor layer and the insulating layer are laminated in advance. In the present invention, the stretchable laminate sheet at least includes the stretchable insulating layer having adhesiveness and the stretchable conductor layer. The stretchable laminate sheet is attached to an inside part of the garment to be in contact with skin for measurement of a biological signal. The stretchable insulating layer having adhesiveness has the 90 degree peel strength against the stretchable conductor layer of 1.0 N/cm or more and 21.0 N/cm or less, so that the stretchable insulating layer supports the stretchable conductor layer under stress and deformation when the garment is put on, taken off, and washed. The stretchable insulating layer thus prevents breakage of chain structure of the electrically conductive fine particles in the stretchable conductor layer upon stretching. Hence, the stretchable insulating layer plays a role in maintaining high conductivity upon repeated stretching.

The stretchable laminate sheet has the tensile elastic modulus of 10 MPa or more and 500 MPa or less, and the load applied to the stretchable laminate sheet at the stretching rate of 10% is 3 N or more and 100 N or less. This allows the stretchable laminate sheet to follow the fabric deformed by postural changes of its wearer, and thus the stretchable laminate sheet does not spoil discomfort. In addition, the stretchable laminate sheet is flexible and stretchable so that it does not spoil a fit of the garment either, even being attached to the garment for use.

Further, the stretchable conductor layer has the sheet resistance of 300 $\Omega_\square$ or less in the absence of stretching and has the sheet resistance increase ratio at the stretching rate of 10% of less than 10, so that the stretchable conductor layer detects an electrical signal necessary for measurement and thus achieves stable electrical signal detection even though the stretchable conductor layer is stretched by deformation of the fabric upon postural changes of a wearer.

The stretchable conductor layer includes the electrically conductive fine particles and the binder resin. The electrically conductive fine particles enables detection of an electrical signal. The binder resin fixes the fine particles and retains a form as the stretchable conductor layer. At this time, the binder resin has the elastic modulus of 1 GPa or less and has the elongation at break of 200% or more. Such a binder resin achieves the stretchable conductor layer having excellent stretchability that follows the fabric and thus does not spoil discomfort. Such a stretchable conductor layer achieves stable electrical signal detection even though the stretchable conductor layer is stretched along with the fabric.

The stretchable insulating layer having adhesiveness, which is laminated with the stretchable conductor layer, includes the flexible resin. However, stretchable conductor layer has a smooth surface that becomes a hindrance for permeation of the flexible resin, and, owing to this hindrance, the flexible resin does not exhibit physical adhesive strength. This deficiency of the physical adhesive strength necessitates chemical adhesive strength for the stretchable insulating layer and the stretchable conductor layer to be stuck and adhere to one another. For this reason, by selecting each resin to meet the following conditions, the 90 degree peel strength between the stretchable insulating layer and the stretchable conductor layer of 1.0 N/cm or more is satisfied: the value calculated by the subtraction of the Hansen solubility parameter value ($\delta$) of the binder resin from the Hansen solubility parameter value ($\delta$) of the flexible resin falls within the range of -5.0 to +5.0 (MPa)$^{0.5}$, and the value calculated by the subtraction of the hydrogen bond term ($\delta h$) of the Hansen solubility parameters of the binder resin from the hydrogen bond term ($\delta h$) of the Hansen solubility parameters of the flexible resin falls within the range of -6.0 to +6.0 (MPa)$^{0.5}$.

The garment for measuring biological information including the stretchable laminate sheets has the 90 degree peel strength between the stretchable laminate sheets and the fabric of 1.0 N/cm or more. When the fabric and the flexible resin are stuck and adhere to one another, the flexible resin permeates into the fabric and thus exhibits physical adhesive strength. This exhibition of the physical adhesive strength achieves, without the resin selection by Hansen solubility parameters, necessary and sufficient adhesive strength that prevents peeling of the stretchable conductor layer when the garment is used or washed.

DESCRIPTION OF EMBODIMENTS

In a garment for measuring biological information of the present invention, a conductor layer for an electrical wire may be metallic foil, a conductive fiber or a conductive thread, a stretchable conductor layer, or the like. The metallic foil may be copper foil, aluminum foil, stainless steel foil, gold foil, or the like. The conductive fiber or the conductive thread may be a fine metal wire, a natural fiber or synthetic fiber with metal coating, a fiber comprised of a polymeric material containing a conductive filler, such as fine metal particles and a carbon nanofiber, a fiber coated with or including a conductive polymer, or the like. The conductor layer for the electrical wire may also be felt, a fabric, a knitted fabric, a non-woven fabric, or the like including the conductive fiber or the conductive thread.

Hereinafter is described a stretchable laminate sheet according to an embodiment of the present invention. The stretchable laminate sheet of the present invention preferably at least includes a stretchable insulating layer having adhesiveness and a stretchable conductive layer. The stretchable laminate sheet is preferably attached to an inside part of the garment via the stretchable insulating layer having adhesiveness. The stretchable insulating layer preferably has a 90 degree peel strength against the stretchable conductor layer of 1.0 N/cm or more and 25.0 N/cm or less.

This 90 degree peel strength between the stretchable insulating layer and the stretchable conductor layer is preferably 1.5 N/cm or more and 15.0 N/cm, and further preferably 2.5 N/cm or more and 10 N/cm or less. A 90 degree peel strength of less than the predetermined range might cause the stretchable conductor layer to peel and drop under stress and deformation when the garment is put on, taken off, and washed. Such a 90 degree peel strength also causes destruction of chain structure of electrically conductive fine particles in the stretchable conductor layer upon stretching, consequently leading to deterioration of conductivity and to disconnection upon repeated stretching. Inversely, a 90 degree peel strength that is more than the predetermined range far exceeds material strength of the stretchable insulating layer or the stretchable conductor layer, thus becoming meaningless in practical terms.

The stretchable laminate sheet has a tensile elastic modulus of 500 MPa or less, and a load applied to the stretchable laminate sheet at a stretching rate of 10% is 100 N or less. When the tensile elastic modulus exceeds 500 MPa or the load applied to the stretchable laminate sheet at the stretching rate of 10% exceeds 100 N, an electrode is hindered from following skin and a poor contact of the electrode is caused by various postures and motions taken by a wearer of the garment. The poor electrode contact results in noise contamination and deterioration of an electrical signal.

The stretchable conductor layer has a sheet resistance of 300 $\Omega_\square$ or less in the absence of stretching and has a sheet resistance increase ratio at a stretching rate of 10% of less than 10. When the sheet resistance in the absence of stretching exceeds 300 $\Omega_\square$ and the sheet resistance increase ratio at a stretching rate of 10% exceeds 10, noise contamination and deterioration of an electrical signal are caused by resistance change upon stretching of a stretchable film layer along with fabric deformation by postural changes of a wearer. The stretchable laminate sheet of the present invention may be used as an electric wire in addition to an electrode for detecting an electrical signal by contact with skin. In the former case, on a front side of the stretchable conductor layer may be further provided a stretchable insulating film layer as an insulating protective layer.

The lower limit of the tensile elastic modulus is approximately 10 MPa. A tensile elastic modulus less than this value excessively weakens material strength, thus becoming impractical.

In the stretchable laminate sheet of the present invention, the stretchable insulating layer having adhesiveness includes a flexible resin. Examples of the flexible resin include a thermoplastic resin, a thermosetting resin, a photocurable resin, and rubber or an elastomer. The thermoplastic resin may be low density polyethylene, an ethylene-vinyl acetate copolymer, polyvinylidene chloride, copolyester, or the like. The thermosetting resin and the photocurable resin may be an acrylic resin, a silicone resin, a polyurethane resin, or the like. Examples of the rubber or the elastomer include urethane rubber, acrylic rubber, silicone rubber, butadiene rubber, nitrile rubber, isoprene rubber, styrene butadiene rubber, butyl rubber, chlorosulfonated polyethylene rubber, ethylene propylene rubber, and a vinylidene fluoride copolymer. Amongst these, preference is given to the rubber or the elastomer for developing stretchability. In addition, the flexible resin may contain water or an organic solvent to enable application or printing.

The stretchable conductor layer having electrical conductivity at least includes electrically conductive fine particles and a binder resin and may have two or more layers each of which has a different component, and different chain structure of the electrically conductive fine particles. The electrically conductive fine particles is fine metal particles and/or carbon fine particles. The fine metal particles may be, for example, metal particles of silver, gold, platinum, palladium, copper, nickel, aluminum, zinc, lead, tin, or the like; alloy particles of brass, bronze, cupronickel, solder, or the like; hybrid particles like silver-coated copper; metal-plated polymer particles; metal-plated glass particles; metal-coated ceramic particles. The carbon fine particles may be graphite powder, activated carbon powder, scaly graphite powder, acetylene black, ketjen black, fullerene, a carbon nanotube, or the like. The electrically conductive fine particles of just one kind, or two or more kinds may be used.

The binder resin preferably has an elastic modulus of 1 GPa or less and has an elongation at break of 200% or more. Example of the binder resin include a thermoplastic resin, a thermosetting resin, a photocurable resin, and rubber or an elastomer. The thermoplastic resin may be low density polyethylene, an ethylene-vinyl acetate copolymer, polyvinylidene chloride, copolyester, or the like. The thermosetting resin and the photocurable resin may be an acrylic resin, a silicone resin, a polyurethane resin, or the like. Examples of the rubber or the elastomer include urethane rubber, acrylic rubber, silicone rubber, butadiene rubber, nitrile rubber, isoprene rubber, styrene butadiene rubber, butyl rubber, chlorosulfonated polyethylene rubber, ethylene propylene rubber, and a vinylidene fluoride copolymer. The binder resins of just one kind, or two or more kinds may be used. Amongst these, preference is given to the rubber or the elastomer for developing stretchability and adhesiveness to the electrically conductive fine particles.

Lamination of the stretchable insulating layer having adhesiveness and the stretchable conductor layer having electrical conductivity, which are preferably stuck to one another, requires chemical adhesive strength in order for the flexible resin and the binder resin, which are included in each layer, to be stuck and adhere to one another. For this reason, in order for the 90 degree peel strength embodied in the present invention to satisfy 1.0 N/cm or more, each resin is preferably selected to meet the following conditions: a value calculated by subtraction of a Hansen solubility parameter value ($\delta$) of the binder resin from a Hansen solubility parameter value ($\delta$) of the flexible resin falls within a range of -5.0 to +5.0 $(MPa)^{0.5}$, and a value calculated by subtraction of a hydrogen bond term ($\delta h$) of Hansen solubility parameters of the binder resin from a hydrogen bond term ($\delta h$) of Hansen solubility parameters of the flexible resin falls within a range of -6.0 to +6.0 $(MPa)^{0.5}$.

The Hansen solubility parameters mentioned here are a solubility index proposed by Dr. Charles M. Hansen et al. In this index, solubility is represented by three parameters: a dispersion term (δd), a polar term (δp) and a hydrogen bond term (δh). Substances having close parameters are judged to have high solubility into one another. The Hansen solubility parameters (δ) are represented by the following equation (1).

$$\delta = ((\delta d)^2 + (\delta p)^2 + (\delta h)^2)^{0.5} \qquad (1)$$

The index represented by these parameters is also applicable to miscibility of resins, and is also useful as an index of the chemical adhesive strength of each resin layer in the stretchable laminate sheet of the present invention. Especially, the selection by the Hansen solubility parameters (δ) and the hydrogen bond term (δh) has been found useful.

A blending amount of the electrically conductive fine particles in the stretchable conductor layer is determined, based upon electrical resistance, stretchability and moisture permeability. The higher volume% of electrically conductive fine particles relative to the binder resin decreases electrical resistance, thus being able to prevent deterioration of an electrical signal. However, such a composition weakens adhesive strength to the stretchable insulating layer and deteriorates launderability and stretchability, thus worsening tightness and a fit. Inversely, the lower volume% of electrically conductive fine particles relative to the binder resin enhances stretchability and improves tightness and a fit. However, such a composition increases electrical resistance, thus deteriorating an electrical signal. To keep a balance between both the properties, the blending amount of the electrically conductive fine particles relative to the binder resin is preferably 20 to 60 volume%.

In the stretchable laminate sheet of the present invention, either or both of the stretchable insulating layer having adhesiveness and the stretchable conductor layer may contain insulating fine particles to a degree that does not spoil necessary properties for exhibiting a mechanical property, heat resistance, durability and moisture permeability. The insulating fine particles is made of an organic or inorganic insulating material. The organic fine particles may be resin fine particles such as acrylic resin fine particles, styrene resin fine particles and melamine resin fine particles. The inorganic fine particles may be ceramic fine particles such as silica, alumina, zirconia, talc, silicon carbide, magnesia, boron nitride; or fine particles of a water-insoluble salt, such as calcium phosphate, magnesium phosphate, barium sulfate and calcium sulfate. The insulating fine particles of just one kind, or two or more kinds may be used.

The stretchable conductor layer of the present invention is produced by a method of, for example, applying or printing conductive paste containing water or an organic solvent in addition to the electrically conductive fine particles and the binder resin. A content of the water or the organic solvent is determined, based, for example, upon a dispersion method of the electrically conductive fine particles, or upon viscosity and a dry method of the conductive paste that are suitable for a production method of a conductive film. The content affects a state of chain structure formation of the electrically conductive fine particles and moisture permeability of a film layer after the application. The content is preferably 20 to 60 mass% when total mass of the electrically conductive fine particles, the binder resin and the solvent is defined to be 100%, but is not particularly limited thereto. A content of less than 20 mass% leads to an increase in viscosity and thus is unsuitable for the application or the printing, consequently causing uneven chain structure of the electrically conductive fine particles and deteriorating conductivity and stretchability. Inversely, a content exceeding 60 mass% leads to an increase in a residual solvent amount in a dry-hardened coat and thus might cause deterioration of reliability of the coat.

The organic solvent preferably has a boiling point of 100°C or more and less than 300°C. An organic solvent having an excessively low boiling point evaporates during a production step of the paste or upon use thereof and thus might be likely to cause variation in a component ratio of the paste. An organic solvent having an excessively high boiling point leads to an increase in a residual solvent amount in a dry-hardened coat and thus might cause reliability deterioration of the coat. Examples of preferable organic solvent include cyclohexanone, toluene, isophorone, γ-butyrolactone, benzyl alcohol, ExxonMobil Chemical's solvesso 100, 150 and 200, propylene glycol monomethyl ether acetate, terpineol, butyl glycol acetate, diamylbenzene (boiling point: 260 to 280°C), triamylbenzene (boiling point: 300 to 320°C), n-dodecanol (boiling point: 255 to 29°C), diethylene glycol (boiling point: 245°C), ethylene glycol monoethyl ether acetate (boiling point: 145°C), diethylene glycol monoethyl ether acetate (boiling point 217°C), diethylene glycol monobutyl ether acetate (boiling point: 247°C), diethylene glycol dibutyl ether (boiling point: 255°C), diethylene glycol monoacetate (boiling point: 250°C), triethylene glycol diacetate (boiling point: 300°C), triethylene glycol (boiling point: 276°C), triethylene glycol monomethyl ether (boiling point: 249°C), triethylene glycol monoethyl ether (boiling point: 256°C), triethylene glycol monobutyl ether (boiling point: 271°C), tetraethylene glycol (boiling point: 327°C), tetraethylene glycol monobutyl ether (boiling point: 304°C), tripropylene glycol (boiling point: 267°C), tripropylene glycol monomethyl ether (boiling point: 243°C), 2,2,4-trimethyl-1,3-pentanediol monoisobutyrate (boiling point: 253°C), and two or more kinds of these organic solvents may be contained as necessary. Such an organic solvent is appropriately contained so that the conductive paste has suitable viscosity for the application or the printing.

To impart application and printing properties, the conductive paste for producing the stretchable conductor layer of the present invention may be blended with a thixotropy imparting agent, a leveling agent, a plasticizer, an antifoaming agent, or the like in a range without impairing necessary properties for a stretchable conductive film. The conductive paste for forming the stretchable conductor layer of the present invention can contain the electrically conductive fine particles dispersed evenly by a conventionally known method for dispersing fine particles in a solution. Such even dispersion can be achieved by, for example, an ultrasonic method, a mixer method, a three roll mill method, a ball mill method, or the like after mixture of a dispersion solution of the fine particles and a solution of the resin. These methods may be employed in combination of plural kinds.

An example method for producing the stretchable laminate sheet of the present invention is as follows. To form a wet coat, the conductive paste is applied or printed on a substrate having mold releasability. The wet coat thus formed is then dried by allowing the organic solvent contained therein to evaporate. Thereafter, a sheet of the stretchable insulating layer having adhesiveness, which has been formed in advance, is stuck to the dried coat. Another example method is as follows. To form a wet coat, the conductive paste is applied or printed on a sheet of the stretchable insulating layer having adhesiveness, which has been formed in advance. Thereafter, the wet coat thus formed is dried by allowing the organic solvent contained therein to evaporate. Yet another example method is as follows. To form a wet coat, the conductive paste is applied or printed on a substrate having mold releasability. The wet coat thus formed is then dried by allowing the organic solvent contained therein to evaporate. Thereafter, on this dried coat is applied or printed a solution of the flexible resin for forming the stretchable insulating layer having adhesiveness. Finally, the wet coat of this flexible resin solution is dried by allowing the organic solvent contained therein to evaporate. Any of these methods may be used. In any of the methods, the film layer, which is prepared in advance, may be subjected to a plasma treatment, a flame treatment, a primer treatment, or the like before a subsequent layer is formed thereon, for the purpose of enhancing adhesive strength between the insulating film layer and the conductive film layer.

An application step of the conductive paste may be performed by, for example, a coating method, a printing method, or the like, but is not particularly limited thereto. Examples of the printing method include a screen printing method, a planographic offset printing method, an inkjet method, a flexographic printing method, a gravure printing method, a gravure offset printing method, a stamping method, a dispensing method and a squeegee printing. A heating and drying step of the applied conductive paste and the applied flexible resin solution may be performed under an atmosphere, a vacuum atmosphere, an inert gas atmosphere, a reducing gas atmosphere, or the like. Such a heating and drying step evaporates the organic solvent and, in some cases, allows a hardening reaction to proceed during the heating, thus yielding favorable electrical resistance and stretchability of a dried stretchable conductor layer. The heating and drying step under an atmosphere is performed at a heating temperature of 50 to 200°C and a heating time of 10 to 90 minutes. A condition for this heating and drying step is selected from either a low temperature-long time combination or a high temperature-short time combination, based upon electrical resistance and stretchability of the stretchable conductor layer, heat resistance of the binder resin, a boiling point of the organic solvent, or the like. A heating temperature of less than 50°C or a heating time of less than 10 minutes causes the solvent to remain in the coat, thus failing to achieve desired electrical resistance and stretchability. A heating temperature exceeding 200°C or a heating time exceeding 90 minutes causes deterioration and a cross-link in the binder resin, the flexible resin or the substrate, thus failing to achieve desired stretchability and leading to cost increase.

The garment for measuring biological information of the present invention includes the stretchable laminate sheets of the present invention that are attached to an inside part of the garment. The substrate of the garment for measuring biological information of the present invention is any band-like object, such as a belt and a brassiere, and/or any garment made of a knitted fabric or a non-woven fabric without any particular limitation. The substrate is, however, preferably a stretchable object from a perspective of, for example, good attachability to a wearer for measurement of biological information, and of followability during exercise and in motion. A 90 degree peel strength between the stretchable laminate sheet and a fabric of the substrate is 1.0 N/cm or more to prevent peeling and disconnection of the electrode, that are caused by stress and deformation when the garment is put on, taken off, and washed. Such a garment for measuring biological information becomes means to measure biological information of its wearer, is put on in a usual manner and offers a usual fit. Hence, just wearing such a garment easily enables measurement of biological information of various kinds.

EXAMPLES

Next, specific examples of the present invention will be described. However, the present invention is not particularly limited to these examples.

[Preparation of Conductive Paste]

With materials shown in Table 1, a resin was dissolved in its corresponding solvent according to a weight blending ratio shown in Table 2. A resultant solution was blended with electrically conductive fine particles and then mixed by a three roll mill to obtain conductive paste.

[Table 1]

| Material | Abbreviation | Contents | $\delta$ (MPa)$^{0.5}$ | $\delta h$ (MPa)$^{0.5}$ |
|---|---|---|---|---|
| Resin | CSM | Chlorosulfonated Polyethylene Rubber CSM-TS530 Available from Tosoh Corporation | 19.9 | 4.1 |
| | DN3 | Nitrile Rubber Nipol DN003 Available from Zeon Corporation | 21,6 | 4.1 |
| | UR61 | Polyester-urethane Resin Vylon UR6100 Available from Toyobo Co., Ltd. | 25.7 | 11.3 |
| Solvent | CHX | Cyclohexanone | - | - |
| | IPH | Isophorone | - | - |
| | SOL | Solvesso#150 | - | - |
| Electrically Conductive Fine Particle | G35 | Aggregated Silver Powder G-35 Available from DOWA Electronics Materials Co., Ltd | - | - |
| | FA3 | Flake-formed Silver Powder FA-D-3 Available from DOW A Electronics Materials Co., Ltd. | - | - |
| Adhesive Resin Solution | 545F | NBR Solvent Adhesive 545F Available from CEMEDINE CO., LTD. (Non-volatile Component of 22 Weight%, Methyl Ethyl Ketone Solution) | 20.1 | 4.3 |
| | UR32 | Polyester-urethane Resin Vylon UR3200 Available from Toyobo Co., Ltd. (Non- volatile Component of 30 Weight%, Methyl Ethyl Ketone/Toluene Mixed Solution) | 24,1 | 11.7 |
| Shirt | Polyester | Cool One 100% Polyester Available from Sanwa Co., Ltd | - | - |

[Table 2]

| Item | | PS01 | PS02 | PS03 |
|---|---|---|---|---|
| Resin [Part by Mass] | CSM | 20 | - | - |
| | DN3 | - | 20 | - |
| | UR6 | - | - | 20 |
| Solvent [Part by Mass] | CHX | - | - | 25 |
| | IPH | - | 45 | - |
| | SOL | 45 | - | 20 |
| Silver particle [Part by Mass] | G35 | 80 | 60 | 60 |
| | FAD3 | - | 20 | 20 |

[Preparation of Stretchable Laminate Sheet]

The conductive paste was applied on a mold release PET film with an applicator in a manner that a dried film thickness of the conductive paste was to become approximately 60 $\mu$m. The resultant film was then dried in a hot air drying oven at 120°C for 30 minutes to form a stretchable conductor layer. Likewise, an adhesive resin solution in Table 1 was applied on another mold release PET film with an applicator in a manner that a dried film thickness of the adhesive resin solution was to become approximately 60 $\mu$m. The resultant film was then dried in a hot air drying oven at 120°C for 30 minutes to form a stretchable insulating layer having adhesiveness. Thereafter, the stretchable conductor layer and the stretchable insulating layer having adhesiveness, both of which had the mold release PET films, were superposed on one another with their mold release PET films facing outwards. The superposed layers were made to adhere to one another with a roll laminating machine whose rubber roll temperature was adjusted to be 120°C to prepare a stretchable laminate sheet having the mold release PET films on its both sides. With these laminated sheets and the below-described methods were measured a thickness, a tensile elastic modulus, a unit load upon 10% stretching, a film electrical resistance in the absence of stretching, a resistance increase ratio upon 10% stretching and a 90 degree peel strength. The measurement results of Examples are shown in Table 3.

[Table 3]

| | | Unit | Example 1 | Example 2 | Example 3 | Comparative Example 1 | Comparative Example 2 | Comparative Example 3 |
|---|---|---|---|---|---|---|---|---|
| Conductive Paste | | - | PS1 | PS2 | PS3 | PS1 | PS2 | PS3 |
| Adhesive Resin | | - | 545F | 545F | UR32 | UR32 | UR32 | 545F |
| Sheet Property | Thickness | $\mu$m | 124 | 122 | 121 | 119 | 130 | 111 |
| | Tensile Elastic Modulus | MPa | 135 | 142 | 139 | 131 | 147 | 120 |
| | Unit Load upon 10% Stretching | N/cm | 70 | 66 | 71 | 75 | 78 | 73 |
| | Sheet Resistance in Absence of Stretching | $\Omega_\square$ | 172 | 182 | 131 | 155 | 174 | 125 |
| | Sheet Resistance Increase Ratio upon 10% Stretching | - | 1.9 | 1.9 | 2.6 | 2.0 | 1.8 | 22 |
| | 90 Degree Peel Strength | N/cm | 2.4 | 1.9 | 2.2 | 0.8 | 0.9 | 0.5 |
| Difference in Hansen Solubility Parameter Values ($\delta$) | | $(MPa)^{0.5}$ | 0.2 | -1.5 | -1.6 | 4.2 | 2.5 | -5.6 |
| Difference in Hydrogen Bond Terms ($\delta$h) of Hansen Solubility Parameters | | $(MPa)^{0.5}$ | 0,2 | 0.2 | 0.4 | 7.6 | 7.6 | -7.0 |
| Washing Durability | Sheet Resistance After 50 Times Washing | $\Omega_\square$ | 310 | 291 | 262 | 729 | 783 | 638 |
| | Sheet Resistance Increase Ratio | - | 1.8 | 1.6 | 2.0 | 4.7 | 4.5 | 5.1 |
| Durability by Putting on and Taking off Shirts | | - | No Peeling | No Peeling | No Peeling | Peeling on One Shirt | Peeling on One Shirt | Breaking of a wire |

[Preparation of Garment Having Electrode and Wire]

The prepared stretchable laminate sheet having the mold release PET films was cut into a 5.0 cm square shaped electrode with a continuous wire having a width of 1.0 cm and a length of 15.0 cm. The release liner on the side of the stretchable insulating layer having adhesiveness was peeled off the electrode with the wire. The electrode with the wire was then disposed on a predetermined position on the back side of a shirt (100% polyester) and thermocompression bonded with an iron. The mold release PET film was lastly peeled off the thermocompression-bonded electrode with the wire to prepare the shirt having the electrode on its back side.

[Measurement of Thickness, Elastic Modulus and Load Applied upon 10% Stretching]

Thickness of a sheet was measured in accordance with JIS K7130 (1999), Plastics-Film and sheeting-Determination of thickness, Method A. An elastic modulus of the sheet and a load applied to the sheet upon stretching were measured in accordance with JIS K7161-1 (2012), Plastics-Determination of tensile properties-Part 1. Measurement conditions were as follows: a specimen width of 15 mm, a specimen length of 150 mm, a distance between chucks of 100 mm and a test speed of 50 mm/min.

[Tensile Test and Measurement of Electrical Sheet Resistance]

The above-prepared stretchable laminate sheet was held with a tensile tester (a manual stretching machine) having two chucks of 2.5 cm in width in a manner that a distance between the chucks became 5.0 cm. The stretchable laminate sheet was then stretched up to a stretching rate of 10% (a displacement of 0.5 cm) in a longitudinal direction thereof. To determine electrical sheet resistances before and after the test, resistance values ($\Omega$) were measured before and after the stretching at outer sides of the two chucks facing one another (a measurement distance: 10 cm) with a Digital Multimeter ("YOKOGAWA TY530" available from Yokogawa Test & Measurement Corporation). The electrical sheet resistances ($\Omega_{\square}$) of the stretchable laminate sheet were determined from the measured values. The measurement of the resistance values was performed immediately after the tensile (within 3 seconds).

[Measurement of 90 Degree Peel Strength]

Adhesive tapes including a substrate having a tensile elastic modulus of 1 GPa or more and a thickness of 20 $\mu$m or more were respectively stuck on a stretchable insulating film layer having adhesiveness and a stretchable film layer having electrical conductivity of a stretchable laminate sheet. Each of the layers was held in conjunction with the adhesive tapes stuck to them. To determine a 90 degree peel adhesive strength of the stretchable laminate sheet, a peel strength between the stretchable insulating film layer having adhesiveness and the stretchable film layer having electrical conductivity was measured in accordance with JIS K6854-1 (1999), Adhesives-Determination of peel strength of bonded assemblies: 90 degree peel. Measurement conditions were as follows: a specimen width of 15 mm, a specimen length of 150 mm and a peel speed of 50 mm/min.
This Example employed the adhesive tapes formed from a polyester film substrate having a substrate thickness of 50 $\mu$m with an adhesive layer having a thickness of 25 $\mu$m thereon.

[Calculation of Hansen Solubility Parameter Values]

A structural formula of each resin was determined from analytical results of H1-NMR, C13-NMR and IR measurement. Using the determined structural formula, Hansen solubility parameter values of each resin were calculated with computer software "Hansen Solubility Parameters in Practice (HSPiP)" in reference to Charles M. Hansen, "Hansen Solubility Parameters; A Users Handbook (CRC Press, 2007)". The calculated values are shown in Table 1.

[Washing Durability]

The above-prepared shirt having the electrode with the wire was washed under a condition in accordance with JIS L 0844. Specifically, the shirt within a laundry net was consecutively washed 5 times with a detergent (Attack available from Kao Corporation) by a mechanical washing machine, and then was once dried in shade. This process was defined as 1 cycle, and this cycle was repeated 10 times. After the washing, sheet resistance of a stretchable conductor sheet was measured, and a difference of the measured value from its initial sheet resistance (the sheet resistance after the washing/the initial sheet resistance) was determined.

[Durability by Putting on and Taking off Shirts]

The above-prepared shirts having the electrodes and the wires were put on and taken off 100 times by 5 male adults as subjects. After this test, visual observations were made on peel states and breakage states of the electrodes and the wires.

As described above, the stretchable laminate sheet of the present invention has sufficient adhesiveness that prevents peeling of the stretchable conductor layer when a garment including the stretchable laminate sheet is put on, taken off, and washed. In addition, a load applied to the stretchable laminate sheet upon stretching is so low that the stretchable laminate sheet does not spoil comfort and a fit of the garment and prevents increase in electrical resistance even upon stretching. This prevention of electrical resistance increase enables detection of a bioelectrical signal with little electrical noise.

INDUSTRIAL APPLICABILITY

The present invention provides a stretchable laminate sheet and a garment for measuring biological information that prevent peeling and breakage of an electrode, which are caused by insufficient adhesion thereof, that provide excellent comfort and an excellent fit, and that enable favorable electrical signal measurement. The stretchable laminate sheet and the garment are applicable to healthcare in a daily life, to a check on biological information during outdoor sports, such as jogging and a marathon, and to labor management of outdoor work on a construction site or the like.

**Claims**

1.  A garment for measuring biological information having an electrical wire comprising:

    a flexible adherend layer having an elongation at break of 10% or more; and
    a stretchable insulating layer having a 90 degree peel strength against the flexible adherend layer of 1.0 N/cm or more and 25.0 N/cm or less.

2.  The garment for measuring biological information according to claim 1,
    wherein the adherend layer is at least one layer selected from the group consisting of a clothing fabric, an insulating base layer, and a conductor layer.

3.  A stretchable laminate sheet at least comprising:

    a stretchable conductor layer used as an electrical wire or a body contact electrode of a garment for measuring biological information; and
    a stretchable insulating layer having a 90 degree peel strength against the stretchable conductor layer of 1.0 N/cm or more and 25.0 N/cm or less.

4.  The stretchable laminate sheet according to claim 3,
    wherein the stretchable laminate sheet has a tensile elastic modulus of 10 MPa or more and 500 MPa or less, and
    wherein a load applied to the stretchable laminate sheet at a stretching rate of 10% is 3 N or more and 100 N or less.

5.  The stretchable laminate sheet according to claim 3 or 4, wherein the stretchable conductor layer has a sheet resistance of 300 $\Omega_\square$ or less in the absence of stretching, and
    wherein the stretchable conductor layer has a sheet resistance increase ratio at a stretching rate of 10% of less than 10.

6.  The stretchable laminate sheet according to any one of claims 3 to 5,
    wherein the stretchable insulating layer having adhesiveness comprises a flexible resin,
    wherein the conductor layer comprises an electrically conductive composition comprising electrically conductive fine particles and a binder resin,
    wherein a value calculated by subtraction of a Hansen solubility parameter value ($\delta$) of the binder resin from a Hansen solubility parameter value ($\delta$) of the flexible resin is -5.0 to +5.0 $(MPa)^{0.5}$, and
    wherein a value calculated by subtraction of a hydrogen bond term ($\delta h$) of Hansen solubility parameters of the binder resin from a hydrogen bond term ($\delta h$) of Hansen solubility parameters of the flexible resin is -6.0 to +6.0 $(MPa)^{0.5}$.

7.  The stretchable laminate sheet according to any one of claims 3 to 6,
    wherein the binder resin has an elastic modulus of 1 GPa or less, and wherein the binder resin has an elongation at break of 200% or more.

8.  A method for producing the stretchable laminate sheet according to any one of claims 3 to 7,
    wherein the stretchable laminate sheet is produced by application or printing.

9.  The garment for measuring biological information according to claim 1 or 2,
    wherein the stretchable laminate sheet according to any one of claims 3 to 8 is used as the electrical wire.

10. The garment for measuring biological information according to claim 9,
    wherein a 90 degree peel strength between the stretchable laminate sheet and a fabric is 1.0 N/cm or more and 25.0 N/cm or less.

## INTERNATIONAL SEARCH REPORT

| | International application No. |
|---|---|
| | PCT/JP2018/036680 |

**A. CLASSIFICATION OF SUBJECT MATTER**
Int.Cl. A61B5/0408(2006.01)i, A61B5/0478(2006.01)i

According to International Patent Classification (IPC) or to both national classification and IPC

**B. FIELDS SEARCHED**

Minimum documentation searched (classification system followed by classification symbols)
Int.Cl. A61B5/04-5/053

| Documentation searched other than minimum documentation to the extent that such documents are included in the fields searched | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>A | WO 2010/029966 A1 (UNIVERSITY OF TSUKUBA) 18 March 2010, paragraphs [0122]-[0157], fig. 10-13 & US 2011/0166491 A1, paragraphs [0147]-[0182], fig. 10-13B | 3-4, 8<br>1-2, 5-7, 9-10 |
| Y | JP 2002-291712 A (SEKISUI PLASTICS CO., LTD.) 08 October 2002, paragraphs [0017]-[0037], fig. 1-8 (Family: none) | 3-4, 8 |
| Y | WO 2016/114298 A1 (TOYOBO CO., LTD.) 21 July 2016, paragraph [0094] & US 2018/0020936 A1, paragraph [0115] | 4, 8 |

☐ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 26 October 2018 (26.10.2018) | 11 December 2018 (11.12.2018) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9215668 A
- JP 2002035141 A
- JP 06185638 B
- WO 2016114339 A

**Non-patent literature cited in the description**

- Hansen Solubility Parameters in Practice (HSPiP). **CHARLES M. HANSEN.** Hansen Solubility Parameters; A Users Handbook. CRC Press, 2007